# EUROPÄISCHE PATENTANMELDUNG

(11) **EP 1 647 235 A2**
(43) Veröffentlichungstag der Anmeldung: **19.04.2006**
(21) Anmeldenummer: 05109594.1
(22) Anmeldetag: 14.10.2005
(51) Int. Cl.: A61B 18/18

(54) **Gerät zur Behandlung von sichtbaren feinen Oberflächenvenen**

(30) Priorität: 14.10.2004 DE 102004050143
(71) Anmelder: EKA Gesellschaft für medizinisch- technische Geräte mbH, 82065 Baierbrunn (DE)
(72) Erfinder: Kreitmaier, Albert, 82065, Baierbrunn (DE)
(74) Vertreter: Kehl, Günther

(57) **Zusammenfassung**

Ein Gerät zur Behandlung von sichtbaren feinen Oberflächenvenen (Besenreiser) weist eine Lichtquelle (1), eine Lichtleitersonde (2) mit einer Behandlungsspitze (3) und ein Steuergerät (4) auf. Die Lichtquelle (1) erzeugt inkohärentes Licht, dessen Intensität und spektrale Verteilung an der Behandlungsspitze (3) so bemessen ist, daß die Oberflächenvenen durch Lichtstrahlung unter weitgehender Schonung der Haut und des benachbarten Gewebes verödet werden.

## Beschreibung

Die Erfindung bezieht sich auf ein Gerät zur Behandlung von sichtbaren feinen Oberflächenvenen (Besenreiser), das eine Lichtquelle, eine Lichtleitersonde mit einer Behandlungsspitze und ein Steuergerät aufweist.

Unter Licht wird hier und im folgenden nicht nur sichtbares Licht, sondern auch die an den Spektralbereich des sichtbaren Lichts angrenzenden Bereiche des Spektrums der elektromagnetischen Strahlung verstanden.

Aus US 6 491 715 B1 ist ein Lasergerät bekannt, mit dem Oberflächenvenen verödet werden können. Vor der Behandlung wird ein Licht absorbierender Farbstoff in die Blutbahn des Patienten injiziert.

Ausgehend von diesem Stand der Technik liegt der Erfindung die Aufgabe zugrunde, ein Gerät der eingangs genannten Art zu schaffen, das sich durch einfache, ungefährliche und wenig zeitraubende Anwendung und kostengünstige Herstellung auszeichnet.

Diese Aufgabe ist dadurch gelöst, daß die Lichtquelle inkohärentes Licht erzeugt, dessen Intensität und spektrale Verteilung an der Behandlungsspitze so bemessen ist, daß die Oberflächenvenen durch Lichtstrahlung unter weitgehender Schonung der Haut und des benachbarten Gewebes verödet werden.

Das Gerät gemäß der Erfindung ist kostengünstig herzustellen, da inkohärente Lichtquellen, wie beispielsweise Glühlampen oder Halogenlampen, wesentlich billiger als kohärente Lichtquellen (Laser) sind. Es wurde gefunden, daß die im Vergleich zu kohärenten Strahlern breite spektrale Verteilung der inkohärenten Strahlung für die Verödung nicht nur nicht störend ist, sondern geradezu wirkungsvoll und nutzbringend zur Verödung der Gefäße eingesetzt werden kann.

Nach einer vorteilhaften Weiterbildung der Erfindung wird durch die räumliche Divergenz der inkohärenten Strahlung im Bereich der Behandlungsspitze sichergestellt, daß in einigem Abstand von der Behandlungsspitze des Geräts, die Intensität der Strahlung bereits so gering ist, daß keine Gefahr einer Verletzung besteht, selbst dann, wenn die Strahlung in das Auge eines Menschen fällt. Das Tragen von Schutzbrillen im Behandlungsraum ist daher nicht erforderlich.

Die Divergenz der Lichtstrahlung beim Austritt aus der Behandlungsspitze kann vorzugsweise zwischen 30° und 120° liegen.

Nach einer besonders vorteilhaften Ausgestaltung der Erfindung weist die Lichtstrahlung eine kontinuierliche Spektralverteilung auf, deren Maximum bei einer Wellenlänge von 0,7 *µ*m bis 2 *µ*m liegt. Es hat sich gezeigt, daß - wie weiter unten noch im Detail anhand eines Beispiels erläutert werden wird - die Strahlung in diesem Spektralbereich, die menschliche Haut sehr gut durchdringt. Das bedeutet, daß die Strahlung die obere Hautschicht ohne größere Wärmeerzeugung durchquert und erst im Bereich der unerwünschten Venen sich in Wärme umwandelt, woraus die gezielte Verödung der Venen resultiert.

Eine Lichtintensität von 20 bis 50 W/cm² im Bereich der Behandlungsspitze führt zu sehr guten Resultaten bei der Venenentfernung bei gleichzeitiger Schonung der Haut und des umliegenden Gewebes.

Nach einer besonders vorteilhaften Ausgestaltung der Erfindung ist die Lichtleitersonde starr ausgebildet ist. Dies erleichtert dem Therapeuten, das Andrücken der Behandlungsspitze auf den Behandlungspunkt.

Es hat sich als besonders vorteilhaft erwiesen, wenn die Lichtleitersonde abgewinkelt ist. Dies ermöglicht dem Therapeuten eine ermüdungsfreie Handhabung des Geräts.

Gemäß einer besonders vorteilhaften Weiterbildung der Erfindung ist die Behandlungsspitze der Lichtleitersonde konvex (ballig). Die Spitze und folglich die Strahlung kann dadurch präzise auf die Versorger von Besenreiserverästelungen gerichtet werden, wodurch diese bei der Bestrahlung besonders effektiv verschlossen werden können.

Nach einer vorteilhaften Ausgestaltung ist die Behandlungsspitze mit einer abnehmbaren Kappe, vorzugsweise aus Polypropylen oder Polyethylen, überdeckt, die vor jeder Behandlung eines Patienten erneuert werden kann, um die Übertragung von Bakterien von einem Patienten zum nächsten zu vermeiden.

Um eine punktgenaue und präzise Behandlung der Oberflächenvenen zu ermöglichen, ist gemäß einer weiteren vorteilhaften Ausgestaltung der Erfindung vorgesehen, daß die Lichtleitersonde im Bereich der Behandlungsspitze einen Kerndurchmesser zwischen 1 mm und 5 mm, vorzugsweise zwischen 2 mm und 4 mm aufweist.

Es wurde festgestellt, daß die (tieferliegenden) Versorger einer Besenreiserverästelung subkutan ausreichend verschlossen werden können, wenn Licht mit Wellenlängen von 0,7 bis 2 *µ*m, vorzugsweise 0,8 bis 1,2 *µ*m und einer Leistungsdichte von 20 bis 50 W/cm², vorzugsweise 30 bis 40 W/cm², eingestrahlt wird, wobei eine Leistungsdichte von 35W/cm² besonders vorteilhaft ist. Besonders gute Ergebnisse wurden erzielt bei Versorgervenen, die 2 bis 3 mm unter der Hautoberfläche liegen, wobei in einem Fall Strahlung mit Wellenlängen von 0,6 bis 2,8 *µ*m und in einem anderen Fall Strahlung mit einer Wellenlänge von 0,7 bis 1,8 *µ*m eingesetzt wurde. Die Strahlungsdosis eines Bestrahlungsvorgangs ist so zu bemessen, daß subkutan eine Temperaturerhöhung von 20 bis 70 ºC erreicht wird. Unter einem Bestrahlungsvorgang wird hier wie im folgenden eine zeitlich zusammenhängende (nicht unterbrochene) Bestrahlung eines einzelnen Körperpunktes verstanden. Die Behandlung eines Patienten besteht im allgemeinen aus vielen solchen einzelnen Bestrahlungsvorgängen, die jeweils nur etwa 0,5 bis 2 Sekunden dauern.

Es hat sich gezeigt, daß die an der Hautoberfläche liegenden Venen besonders wirksam mit vergleichsweise kurzwelliger Strahlung im Bereich von 0,4 bis 0,8 *µ*m, vorzugsweise von 0,4 bis 0,8 *µ*m, verödet werden. Bei der Therapie wird daher bevorzugt in zwei Phasen gearbeitet, einer ersten Phase, bei der die tieferliegenden Versorgervenen mit relativ langwelliger Strahlung und danach in einer zweiten Phase die an der Oberfläche liegenden Venen mit einer vergleichsweise kurzwelligen Strahlung behandelt werden.

Auf der Hautoberfläche soll die Temperaturerhöhung möglichst klein sein, wie etwa 10 ºC bis maximal 50 ºC, vorzugsweise kleiner 30 ºC sein. Durch Kühlung der Oberfläche vor der Bestrahlung, beispielsweise durch Eis, oder während der Bestrahlung durch gekühlte Luft, kann eine Temperaturerhöhung an der Hautoberfläche auch gänzlich vermieden werden.

Gemäß einer besonders vorteilhaften Ausgestaltung der Erfindung ist die Strahlungsdosis eines zusammenhängenden Bestrahlungsvorgangs an der Behandlungsspitze 0,05 bis 0,5 Joule pro Quadratmillimeter Behandlungsfläche. Mit dieser Lichtdosis läßt sich subkutan (2 bis 3 mm unterhalb der Oberfläche) innerhalb von einigen Zehntel Sekunden eine Temperaturerhöhung bis zu 40ºC erreichen, ohne daß die gekühlte Oberfläche eine merkliche Temperaturerhöhung erfährt. An der Oberffläche bleibt die Gewebetemperatur deutlich unter der Koagulationsgrenze, während im subkutanen Bereich mit einer Temperatur von 50 ºC bis 70 ºC Koagulation (sogar irreversibel) stattfindet.

Es ist auch möglich, die gesamte Strahlungsdosis eines zusammenhängenden Bestrahlungsvorgangs an der Behandlungsspitze zwischen 0,5 bis 5 Joule einzustellen.

Gemäß einer besonders bevorzugten Ausgestaltung der Erfindung ist das Steuergerät so ausgelegt, daß die Lichtstrahlung nur für eine begrenzte Bestrahlungszeit kontinuierlich erzeugt wird.

Die Bestrahlungszeit ist vorzugsweise einstellbar.

Das Steuergerät ist vorzugsweise so ausgelegt ist, daß die Lichtstrahlung für eine Bestrahlungszeit von höchstens 2 Sekunden, vorzugsweise von höchstens 1 Sekunde, kontinuierlich erzeugt wird.

Für die praktische Handhabung hat sich ein Gerät als besonders vorteilhaft erwiesen, bei dem die Lichtquelle in einem Handstück angeordnet ist.

Das Handstück kann mindestens einen Bedienschalter aufweisen, über den die Bestrahlungszeit vorwählbar ist. Wenn der Therapeut eine Änderung der Bestrahlungsdauer vornehmen will, muß er hierzu das Handstück nicht zur Seite legen, vielmehr kann er nach einem Bestrahlungsvorgang augenblicklich die Bestrahlungszeit umstellen.

Eine weitere Ausgestaltung der Erfindung sieht vor, daß das Handstück mindestens einen Handtaster zum Auslösen eines Bestrahlungsvorgangs aufweist.

Erfindungsgemäß wurde erkannt, daß die spektrale Zusammensetzung des Lichts einer Glühlampe, insbesondere einer Halogenlampe, sich generell sehr gut für die Behandlung von Besenreisern eignet. Falls es dennoch in bestimmten Anwendungsfällen, wie beispielsweise bei sehr stark pigmentierter Haut, erforderlich sein sollte, Licht aus bestimmten Teilen des Spektrums zu unterdrücken, kann vorteilhaft ein Filter eingesetzt werden.

Je nach den Umständen des Falles kann ein Bandfilter oder ein Kantenfilter eingesetzt werden, das vorzugsweise im Wellenlängenbereich von 0,7 bis 1,5 *µ*m durchlässig ist.

Durch auswechselbare Filter kann das Gerät an die Bedürfnisse des Patienten angepaßt werden. Insbesondere ist es möglich, unterschiedliche spektrale Lichtzusammensetzungen für verschiedene Zwecke einzusetzen (beispielsweise Verödung von tiefliegenden Versorgervenen oder Verödung von an der Oberfläche liegenden Venen), wobei ein rascher Wechsel möglich ist.

Gemäß einer vorteilhaften Ausgestaltung der Erfindung weist das Steuergerät einen Betriebsstundenzähler auf. Mit diesem kann nicht nur die Brenndauer der Lichtquelle bestimmt werden, um diese rechtzeitig zu ersetzen, vielmehr kann auch der Umfang der Gerätebenutzung erfaßt werden, was bei Mietgeräten, bei Mietkaufgeräten oder für die betriebswirtschaftliche Kalkulation von Bedeutung ist.

Die Erfindung wird im folgenden anhand eines schematisch dargestellten Ausführungsbeispiels näher erläutert.
Figur 1 zeigt im Längsschnitt ein Handstück des erfinungsgemäßen Geräts;
Figur 2 zeigt eine Lichtleitersonde, die an dem Handstück zu befestigen ist;
Figur 3 zeigt das vollständige Gerät mit Handstück, Lichtleitersonde und Steuergerät in perspektivischer Darstellung;
Figur 4 ist ein Schaubild, das die Durchlässigkeit der menschlichen Haut, in Abhängigkeit von der Wellenlänge des Bestrahlungslichts, für vier verschiedene Tiefen zeigt;
Figur 5 zeigt das Strahlungsspektrum der Lichtquelle;
Figur 6 eine Kurve, die das Einsetzen der Koagulation in Abhängigkeit der Temperatur und der Einwirkungszeit t veranschaulicht.

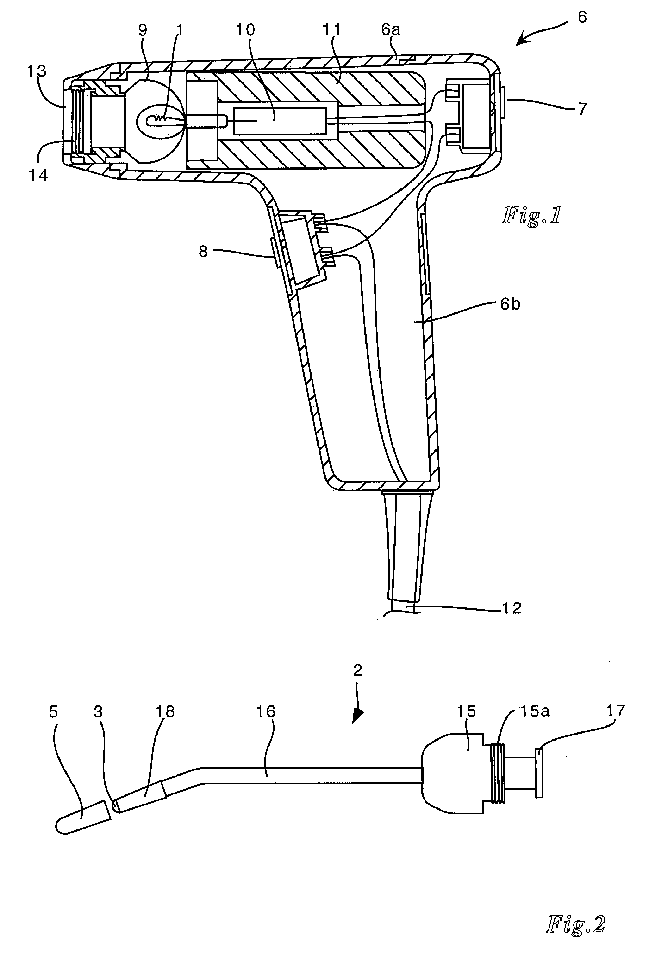

In Figur 1 ist das Handstück 6 des erfindungsgemäßen Geräts zur Behandlung von sichtbaren feinen Oberflächenvenen zu erkennen. Das Gehäuse des Handstücks 6 umfaßt einen rohrförmigen Teil 6a und einen Pistolengriff 6b. In dem rohrförmigen Teil 6a ist eine Lichtquelle in Form einer Wolfram-Halogen-Lampe 1 und ein die Lichtquelle 1 halbseitig umgebender Reflektor 9 konzentrisch zu dem rohrförmigen Teil 6a angeordnet.

In dem rohrförmigen Teil 6a ist ferner eine Lampenfassung 10 und ein Kühlkörper 11 zu erkennen. Im Pistolengriff 6b ist ein Handtaster 8 angeordnet, der durch den Zeigefinger einer den Pistolengriff umfassenden Hand betätigt werden kann, um den Bestrahlungsvorgang auszulösen. Ein Schalter 7 an der Rückseite des rohrförmigen Teils 6a dient zum Einstellen der Bestrahlungszeit eines Bestrahlungsvorgangs. Der Schalter 7 umfaßt zwei Taster (in der Figur nicht zu erkennen), mit denen die Bestrahlungszeit erhöht oder erniedrigt werden kann. Die Lampenfassung 10, der Schalter 7 und der Handtaster 8 sind über ein nach außen geführtes Kabel mit einem Steuergerät (in Figur 1 nicht dargestellt) verbunden, das weiter unten näher erläutert wird.

Das rohrförmige Teil 6a ist an der Stirnseite, in deren Nähe sich die Lichtquelle 1 befindet, offen. Die Gehäuseöffnung 13 dient zur Aufnahme einer Lichtleitersonde 2 (Figur 2). In der Gehäuseoffnung 13 ist ein Innengewinde 14 vorgesehen, in das ein entsprechendes Gewinde 15a einer Befestigungsschraube 15 der Lichtleitersonde 2 (Figur 2) eingeschraubt werden kann.

Figur 2 zeigt die Lichtleitersonde 2. Die Lichtleitersonde 2 weist einen ummantelten Quartzglas-Stab 16 auf, der mit einem Endflansch 17 versehen ist. Der Endflansch 17 ist so dimensioniert, daß er in die Gehäuseöffnung 13 des Handstücks 6 (Figur 1) eingefügt werden kann. Eine in bezug auf den Quartzglas-Stab 16 drehbare Befestigungsschraube 15 mit einem Gewinde 15a dient zur Befestigung der Lichtleitersonde 2 in der Gehäuseöffnung 13 des Handstücks 6. Das dem Endflansch gegenüberliegende Ende des Quartzglas-Stabs 16 stellt die Behandlungsspitze 3 dar. Wie in der Zeichnung zu erkennen ist, ist die Behandlungsspitze 3 konvex ausgeformt. In der Nähe der Behandlungsspitze 3 ist der Quartzglas-Stab 16 mit einer Metallhülse 18 ummantelt. Der Quartzglas-Stab 16 ist nahe der Behandlungsspitze um etwa 30º abgewinkelt, wodurch die Handhabung durch den Therapeuten wesentlich erleichtert wird. Die Behandlungsspitze 3 und der angrenzende Bereich der Metallhülse 18 ist mit einer auswechselbaren Kappe 5 aus Polypropylen überzogen. Wenn die Lichtleitersonde 2 in die Öffnung 13 des Handstücks 6 eingesetzt und mit der Befestigungsschraube 15 festgezogen ist, befindet sich die Stirnkante des Quartzglas-Stabs 16 (in der Zeichnung nicht zu sehen) im optischen Brennpunkt der aus der Lichtquelle 1 und dem hohlspiegelförmigen Reflektor 9 bestehenden Beleuchtungseinrichtung.

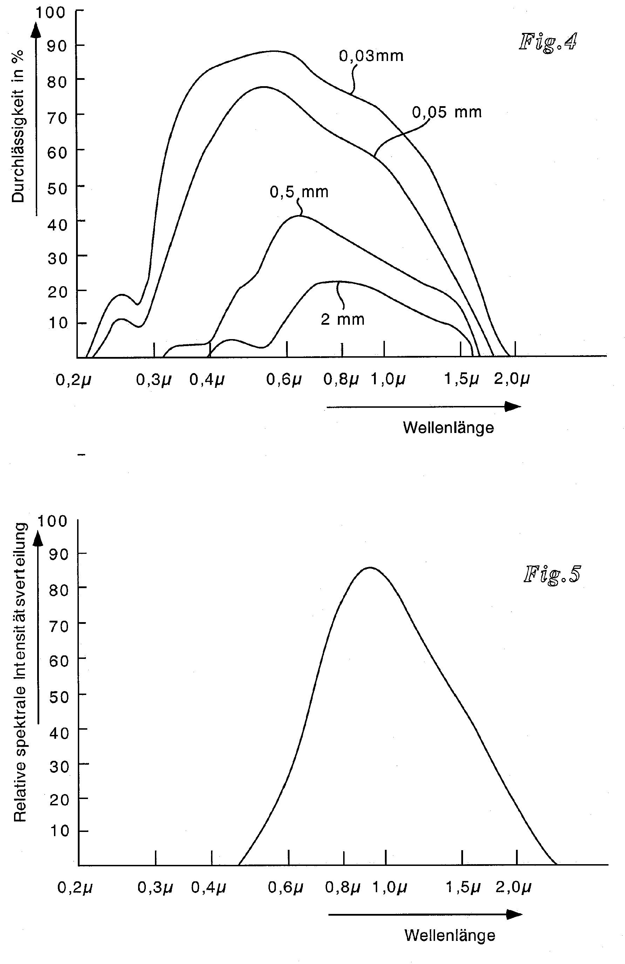

Figur 3 zeigt das vollständige Gerät zur Behandlung von sichtbaren feinen Oberflächenvenen. Die Hauptteile des Geräts sind: das Handstück 6a, 6b mit eingeschraubter Lichtleitersonde 2 und die Steuereinheit 4. Das Handstück 6 ist über das Verbindungskabel 12 mit der Steuereinheit 4 verbunden. Die an das Haushaltsstromnetz angeschlossene Steuereinheit 4 (Anschluß an das Stromnetz nicht gezeigt) liefert über das Verbindungskabel 12 den zum Betrieb der Lichtquelle erforderlichen Strom.

Die Steuereinheit 4 enthält einen Rückwärtszähler, dessen Anfangswert durch Betätigung des Schalters 7 ("+", "-") am Handstück 6 eingestellt werden kann. Die Einstellung kann an der Anzeige 19 überprüft werden. Durch Drücken des Handtasters 8 am Pistolengriffteil 6b wird die Steuereinheit 4 veranlaßt die Stromzufuhr zur Lichtquelle 1 herzustellen. Gleichzeitig wird der Rückwärtszähler in der Steuereinheit in Gang gesetzt. Sobald der Rückwärtszähler den Zählwert Null erreicht hat, wird die Stromzufuhr in die Lichtquelle unterbrochen und der Rückwärtszähler auf seinen Anfangswert zurückgestellt. Der Bestrahlungsvorgangs dauert in der Regel weniger als 1 Sekunde, maximal 2 Sekunden.

Es ist gewährleistet, daß ein einzelner Bestrahlungsvorgang nicht länger als die eingestellte Zeit dauern kann. Nach Ablauf der Zeit setzt der Therapeut die Behandlungsspitze 3 an einem anderen Behandlungsort an und betätigt erneut den Handtaster 8 zur Auslösung eines neuen Bestrahlungsvorgangs.

Figur 4 zeigt ein Schaubild, das die Durchlässigkeit der menschlichen Haut, in Abhängigkeit von der Wellenlänge des Bestrahlungslichts, für vier verschiedene Tiefen, nämlich für eine Tiefe von 0,03 mm, 0,05 mm, 0,5 mm und 2 mm zeigt. Für die Abszisse, an der die Wellenlänge aufgetragen ist, ist eine logarithmische Einteilung gewählt.

Wie Figur 4 zeigt, sind die oberen Hautschichten nahezu voll durchlässig, was bedeutet, daß die Strahlung ohne nennenswerte Verluste und ohne wesentliche Erwärmung der oberen Schichten dieselben durchdringt. Die Absorption des Lichts und damit die Erwärmung findet im wesentlichen in den tieferen Schichten (einige mm Tiefe) statt, in denen üblicherweise Besenreiser anzutreffen sind.

Figur 5 zeigt das Strahlungsspektrum der Lichtquelle. Für die Abszisse, an der die Wellenlänge aufgetragen ist, ist die gleiche logarithmische Einteilung wie in Figur 4 gewählt. Wie aus der Zusammenschau der beiden Diagramme der Figuren 4 und 5 zu ersehen ist, ist die Durchlässigkeit der Haut, insbesondere der oberen Hautschichten, für den Spektralbereich, in dem die Lichtquelle das meiste Licht emittiert, groß, so daß der überwiegende Anteil des ausgesandten Lichts die oberen Hautschichten ohne wesentliche Erwärmung durchdringen und an die Oberflächenvenen gelangen kann, um diese zu veröden.

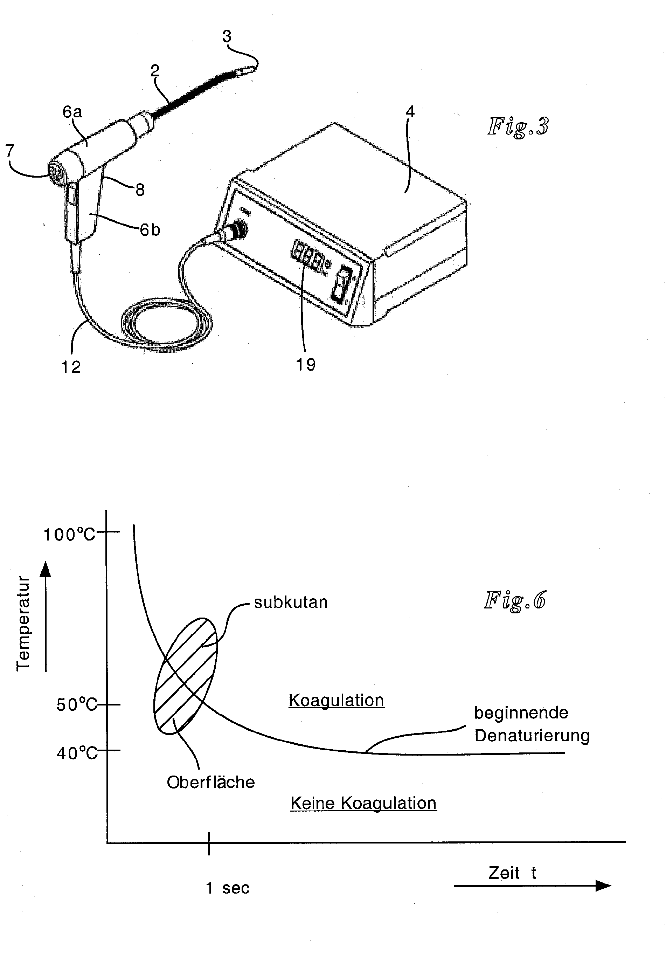

Figur 6 zeigt eine Kurve, die das Einsetzen der Koagulation (beginnende Denaturierung des Proteins) in Abhängigkeit der Temperatur und der Einwirkungszeit t veranschaulicht. Der Bereich oberhalb der Kurve stellt den Bereich dar, in dem Koagulation stattfindet. Unterhalb derselben findet keine Koagulation statt. Wie dem Diagramm zu entnehmen ist, findet etwas unterhalb einer Temperatur von 40 ºC keine Koagulation (auch nicht nach langer Einwirkungszeit) statt. Bei einer Temperatur von 50 ºC beginnt die Koagulation nach knapp einer Sekunde. Ab 70ºC ist die Denaturierung irreversibel. Durch Einbringen einer Lichtdosis von 3 bis 4 Joule, vorzugsweise 3,5 Joule, läßt sich subkutan (2 bis 3 mm unterhalb der Oberfläche) innerhalb von einigen Zehntel Sekunden eine Temperaturerhöhung bis zu 40ºC erreichen, ohne daß die gekühlte Oberfläche eine merkliche Temperaturerhöhung erfährt. Dieser Arbeitsbereich ist durch die schraffierte Fläche markiert. An der Oberffläche bleibt die Gewebetemperatur deutlich unter der Koagulationsgrenze, während der subkutane Bereich mit einer Temperatur von 50 ºC bis 70 ºC im Koagulationsbereich liegt.

Alle beschriebenen Merkmale des vorgestellten bevorzugten Ausführungsbeispiels können mit jedem beliebigen anderen beschriebenen Merkmal oder mit einer beliebigen Gruppe von anderen beschriebenen Merkmalen erfolgreich kombiniert werden, sofern in der Beschreibung im Einzelfall nichts gegenteiliges ausgesagt ist.

## Patentansprüche

1. Gerät zur Behandlung von sichtbaren feinen Oberflächenvenen (Besenreiser), das eine Lichtquelle (1), eine Lichtleitersonde (2) mit einer Behandlungsspitze (3) und ein Steuergerät (4) aufweist, **dadurch gekennzeichnet, daß** die Lichtquelle (1) inkohärentes Licht erzeugt, dessen Intensität und spektrale Verteilung an der Behandlungsspitze (3) so bemessen ist, daß die Oberflächenvenen durch Lichtstrahlung unter weitgehender Schonung der Haut und des benachbarten Gewebes verödet werden.

2. Gerät nach Anspruch 1, bei dem die Lichtquelle (1) eine Glühlampe ist.

3. Gerät nach einem der vorhergehenden Ansprüche, bei dem die Lichtquelle (1) eine Halogenlampe ist.

4. Gerät nach einem der vorhergehenden Ansprüche, bei dem die Lichtstrahlung an der Behandlungsspitze (3) divergent ist.

5. Gerät nach einem der vorhergehenden Ansprüche, bei dem die Lichtstrahlung an der Behandlungsspitze (3) einen Divergenzwinkel von 30 bis 120º aufweist.

6. Gerät nach einem der vorhergehenden Ansprüche, bei dem die Lichtstrahlung eine kontinuierliche Spektralverteilung aufweist.

7. Gerät nach einem der vorhergehenden Ansprüche, bei dem die Lichtstrahlung eine kontinuierliche Spektralverteilung aufweist, deren Maximum bei einer Wellenlänge von 0,7 *µ*m bis 2 *µ*m liegt.

8. Gerät nach einem der vorhergehenden Ansprüche, bei dem die Lichtquelle (1) so ausgelegt ist, daß im Bereich der Behandlungsspitze (3) die Lichtintensität 20 bis 50 W/cm² beträgt.

9. Gerät nach einem der vorhergehenden Ansprüche, bei dem die Lichtleitersonde (2) starr ausgebildet ist.

10. Gerät nach einem der vorhergehenden Ansprüche, bei dem die Lichtleitersonde (2) abgewinkelt ist.

11. Gerät nach einem der vorhergehenden Ansprüche, bei dem die Behandlungsspitze (3) der Lichtleitersonde (2) konvex ist.

12. Gerät nach einem der vorhergehenden Ansprüche, bei dem die Behandlungsspitze (3) mit einer abnehmbaren Kappe (5) überdeckt ist.

13. Gerät nach Anspruch 10, bei dem die abnehmbare Kappe (5) aus Polypropylen oder Polyethylen gefertigt ist.

14. Gerät nach einem der vorhergehenden Ansprüche, bei dem die Lichtleitersonde (2) im Bereich der Behandlungsspitze (3) einen Kerndurchmesser zwischen 1 mm und 5 mm, vorzugsweise zwischen 2 mm und 4 mm aufweist.

15. Gerät nach einem der vorhergehenden Ansprüche, bei dem die Strahlungsdosis eines zusammenhängenden Bestrahlungsvorgangs so bemessen ist, daß subkutan eine Temperaturerhöhung von 20 bis 70 ºC erreicht wird.

16. Gerät nach einem der vorhergehenden Ansprüche, bei dem die Strahlungsdosis eines zusammenhängenden Bestrahlungsvorgangs so bemessen ist, daß auf der Hautoberfläche eine Temperaturerhöhung von 10 ºC bis maximal 50 ºC, vorzugsweise kleiner 30 ºC erreicht wird.

17. Gerät nach einem der vorhergehenden Ansprüche, bei dem die Strahlungsdosis eines zusammenhängenden Bestrahlungsvorgangs an der Behandlungsspitze 0,05 bis 0,5 Joule pro Quadratmillimeter Behandlungsfläche ist.

18. Gerät nach einem der vorhergehenden Ansprüche, bei dem die gesamte Strahlungsdosis eines zusammenhängenden Bestrahlungsvorgangs an der Behandlungsspitze zwischen 0,5 bis 5 Joule ist.

19. Gerät nach einem der vorhergehenden Ansprüche, bei dem das Steuergerät (4) so ausgelegt ist, daß die Lichtstrahlung nur für eine begrenzte Bestrahlungszeit kontinuierlich erzeugt wird.

20. Gerät nach Anspruch 19, bei dem die Bestrahlungszeit einstellbar ist.

21. Gerät nach Anspruch 19 oder 20, bei dem das Steuergerät (4) so ausgelegt ist, daß die Lichtstrahlung für eine Bestrahlungszeit von höchstens 2 Sekunden, vorzugsweise von höchstens 1 Sekunde, kontinuierlich erzeugt wird.

22. Gerät nach einem der vorhergehenden Ansprüche, bei dem die Lichtquelle (1) in einem Handstück (6) angeordnet ist.

23. Gerät nach Anspruch 22, bei dem das Handstück (6) mindestens einen Bedienschalter (7) enthält, über den die Bestrahlungszeit vorwählbar ist.

24. Gerät nach Anspruch 18 oder 19, bei dem das Handstück (6) mindestens einen Handtaster (8) zum Auslösen eines Bestrahlungsvorgangs aufweist.

25. Gerät nach einem der vorhergehenden Ansprüche, das ein Spektralfilter aufweist.

26. Gerät nach Anspruch 25, bei dem das Spektralfilter ein Bandfilter ist.

27. Gerät nach Anspruch 26, bei dem das Spektralfilter im Wellenlängenbereich von 0,7 bis 1,5 *µ*m durchlässig ist.

28. Gerät nach Anspruch 26, bei dem das Spektralfilter im Wellenlängenbereich von 0,6 bis 2,8 *µ*m, vorzugsweise von 0,7 bis 1,8 *µ*m durchlässig ist.

29. Gerät nach Anspruch 26, bei dem das Spektralfilter im Wellenlängenbereich von 0,4 bis 0,8 *µ*m, vorzugsweise von 0,4 bis 0,7 *µ*m durchlässig ist.

30. Gerät nach einem der vorhergehenden Ansprüche, bei dem ein kühlbares, wärmeleitendes und lichtdurchlässiges Haut-Kontakt-Plättchen, vorzugsweise ein mit einem Peltierelement verbundenes Saphirplättchen mit reflexionsvermindernden Oberflächenschichten, vorgesehen ist.

31. Gerät nach einem der vorhergehenden Ansprüche, bei dem das Steuergerät (4) einen Betriebsstundenzähler enthält.
